# EUROPÄISCHE PATENTSCHRIFT

(11) **EP 3 239 395 B1**
(45) Veröffentlichungstag und Bekanntmachung des Hinweises auf die Patenterteilung: **08.05.2019**
(21) Anmeldenummer: 17157480.9
(22) Anmeldetag: 22.02.2017
(51) Int. Cl.: D21F 7/00, G01N 33/34

(54) **VORRICHTUNG ZUR MESSUNG DER FEUCHTIGKEIT DES AUF EINEM SIEBBAND BEFINDLICHEN PULPEMATERIALS**
DEVICE FOR MEASURING THE HUMIDITY OF PULP MATERIAL LOCATED ON A FILTERING BELT
DISPOSITIF DE MESURE DE L'HUMIDITÉ D'UNE PÂTE SE TROUVANT SUR UN TAMIS À BANDE

(30) Priorität: 25.04.2016 AT 2112016
(43) Veröffentlichungstag der Anmeldung: 01.11.2017
(73) Patentinhaber: Bartelmuss, Klaus, 8833 Teufenbach (AT)
(72) Erfinder: Bartelmuss, Klaus, 8833 Teufenbach (AT)
(74) Vertreter: Beer & Partner Patentanwälte KG

(56) Entgegenhaltungen:
- EP-A1- 1 517 130
- EP-A2- 0 303 356
- EP-A2- 1 521 075
- JP-A- S 593 248
- US-A- 5 313 167

## Beschreibung

Die gegenständliche Erfindung betrifft einen Messstab mit einer Einrichtung zur Messung der Feuchtigkeit des auf einem Siebband in einer Anlage zur Erzeugung von Papier befindlichen Pulpematerials, welche einen Feuchtigkeitssensor aufweist, mit einer dieser zugeordneten Steuer- und Datenverarbeitungseinheit sowie mit einem Sende- und Empfangsgerät und bzw. oder mit einem Anschluss für eine Datenleitung zur Übermittlung der Messdaten an die Steuer- und Datenverarbeitungseinheit.

Es ist bekannt, in einer Anlage zur Erzeugung von Papier die Feuchtigkeit des auf dem Siebband befindlichen Pulpematerials zu messen und die hierdurch ermittelten Messwerte zur Steuerung des Betriebes dieser Anlage zu verwenden. Diese Messungen werden an unterschiedlichen Stellen über die Länge des Siebbandes und in einem Abstand von etwa einem halben Meter bis zu einem Meter von einem der Seitenränder des Siebbandes vorgenommen. Die an den einzelnen Messstellen ermittelten Werte der Feuchtigkeit des Pulpematerials werden in einen Datenspeicher eingegeben.

Für die Durchführung dieser Messungen werden Messstäbe verwendet, welche an einem ihrer beiden Enden mit einer Einrichtung zur Messung der Feuchtigkeit des am Siebband befindlichen Pulpematerials ausgebildet sind. Um die Messungen durchführen zu können, wird die Messeinrichtung mittels des Messstabes an vorgegebenen Messstellen an die Unterseite des Siebbandes angepresst.

Betreffend diesen bekannten Stand der Technik wird z.B. auf die EP 2 162 731 B1 verwiesen.

Aus JPS59 3248 A ist eine Vorrichtung zur Messung der Feuchtigkeit des auf einem Siebband zur Erzeugung von Papier befindlichen Pulpematerials bekannt, bei der der Sensor bei der Messung mit einem vorgegebenen Druck an das Siebband gedrückt wird.

Durch den aus EP 2 162 731 B1 bekannten Stand der Technik wird dem Erfordernis einer optimalen Steuerung des Trocknungsverfahrens deshalb nicht entsprochen, da bei diesen Messungen derjenige Druck, mit welchem die Messeinrichtung an das Siebband angepresst wird, nicht berücksichtigt wird.

Der gegenständlichen Erfindung liegt somit die Aufgabe zugrunde, einen Messstab zu schaffen, durch welche dieser Nachteil vermieden wird.

Diese Aufgabe wird erfindungsgemäß dadurch gelöst, dass bei der Messung der Feuchtigkeit derjenige Druck ermittelt wird, mit welchem die Einrichtung zur Messung der Feuchtigkeit an die Unterseite des Siebbandes angepresst wird und die Messung der Feuchtigkeit in einem vorgegebenen Bereich dieses Anpressdruckes erfolgt.

Beim erfindungsgemäßen Messstab ist die Einrichtung zur Messung der Feuchtigkeit des Pulpematerials mit einem Messgerät zur Ermittlung und Anzeige desjenigen Druckbereiches, mit welchem die Einrichtung zur Messung der Feuchtigkeit des Pulpematerials an das Siebband angepresst wird, ausgebildet.

Vorzugsweise ist die Einrichtung zur Messung der Feuchtigkeit mit einem Gehäuse ausgebildet, in welchem ein Feuchtigkeitssensor, welcher unter Wirkung eines Federelementes steht, verstellbar ist. Insbesondere ist das Gehäuse mit einem zylindrischen Hohlraum ausgebildet, in welchem sich der Feuchtigkeitssensor befindet und in welchem er unter Wirkung des Federelementes verschiebbar ist. Vorzugsweise ist das Federelement durch eine Schraubendruckfeder gebildet, welche zwischen dem Feuchtigkeitssensor und dem Gehäuse wirksam ist.

Dabei kann der im Gehäuse befindliche zylindrische Hohlraum mit einem im Querschnitt ringförmigen Bereich ausgebildet sein und kann der Feuchtigkeitssensor mit einer an diesem befestigten Gleithülse ausgebildet sein, welche in den ringförmigen Bereich hineinragt und zwischen dessen Stirnflächen verschiebbar ist. Insbesondere befindet sich das Federelement zwischen der Gleithülse und dem Gehäuse.

Vorzugsweise ist der Feuchtigkeitssensor an seiner dem Siebband zugewandten Seite mit einer verschleißfesten Auflage, insbesondere einer Platte aus einem keramischen Material, ausgebildet, welche am Feuchtigkeitssensor auswechselbar befestigt ist. Weiters kann zwischen der Auflage aus keramischem Material und dem Feuchtigkeitssensor eine Dichtung vorgesehen sein. Zudem sind vorzugsweise der Feuchtigkeitssensor und das Gehäuse mittels einer elastischen Dichtungsmanschette und gegebenenfalls mittels mindestens eines Dichtungsringes gegenüber dem Eintritt von Flüssigkeit abgedichtet.

Vorzugsweise ist der Gleithülse mindestens ein Lagesensor zugeordnet, durch welchen die Stellung des Feuchtigkeitssensors gegenüber dem Gehäuse ermittelt und angezeigt wird. Hierfür kann die Gleithülse mit zwei voneinander in axialem Abstand befindlichen Gleitringen ausgebildet sein, welche mit dem Lagesensor zusammenwirken. Weiters kann an den Lagesensor eine vorzugsweise im Gehäuse befindliche LED-Leuchte angeschlossen sein, durch welche ein Lichtsignal abgegeben wird, sobald sich durch eine Verschiebung des Feuchtigkeitssensors gegenüber dem Gehäuse der Lagesensor in dem zwischen den Gleitringen befindlichen Bereich befindet.

Vorzugsweise sind das Gehäuse der Messeinrichtung und der Messstab mit einander zugeordneten Kupplungsteilen zu deren mechanischer und elektrischer Verbindung ausgebildet. Dabei sind vorzugsweise die Kupplungsteile mit einander zugeordneten Platinen und Zentrierelementen ausgebildet. Zudem kann der Messstab in seiner Länge ein- und feststellbar sein.

Eine erfindungsgemäße Vorrichtung sind nachstehend anhand von in der Zeichnung dargestellten Ausführungsbeispielen näher erläutert. Es zeigen:
- FIG.1: eine Vorrichtung zur Messung der Feuchtigkeit des auf einem Siebband in einer Anlage zur Erzeugung von Papier befindlichen Pulpematerials, in axonometrischer Darstellung,
- FIG.2,: FIG.2A, FIG.2B, FIG.2C die einzelnen Bestandteile der Vorrichtung gemäß FIG.1, in axonometrischer Darstellung,
- FIG.3: eine Einrichtung zur Messung der Feuchtigkeit des auf einem Siebband befindlichen Pulpematerials, welche einen Bestandteil der Vorrichtung nach FIG.1 bildet, in einer ersten Lage einer Einrichtung zur Ermittlung des Anpressdruckes und in vertikalem Schnitt, sowie
- FIG.3A: die Einrichtung gemäß FIG.3 in einer zweiten Lage der Einrichtung zur Ermittlung des Anpressdruckes und in vertikalem Schnitt.

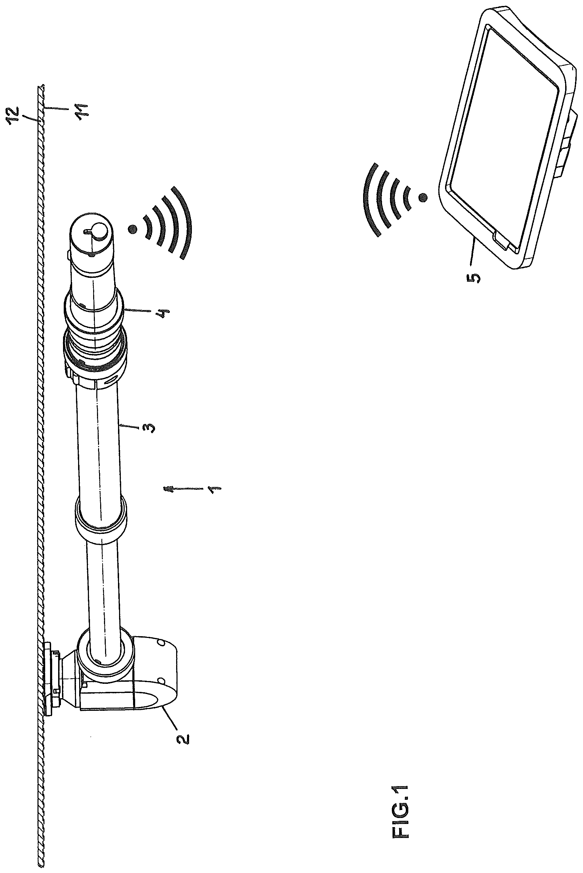

Die in FIG.1 dargestellte Vorrichtung 1 zur Messung der Feuchtigkeit des auf einem Siebband 11 in einer Anlage zur Erzeugung von Papier befindlichen Pulpematerials 12 weist eine Messeinrichtung 2, einen Messstab 3, einen Griffteil 4 und eine Steuer- und Datenverarbeitungseinheit 5 auf.

Zur Durchführung der Messungen wird die Messeinrichtung 2 mittels des Messstabes 3 an die Unterseite des Siebbandes 11, auf dessen Oberseite sich das Pulpematerial 12 befindet, angepresst. Diese Messungen werden an vorgegebenen Stellen über die Länge des Siebbandes 11 und im Abstand von etwa einem halben Meter bis zu einem Meter von einem der Ränder des Siebbandes 11 vorgenommen. Der Messstab 3 ist in seiner Länge ein- und feststellbar. Im Griffteil 4 befinden sich die erforderlichen elektrischen und elektronischen Einrichtungen zur Durchführung und Steuerung der Messungen. Insbesondere befindet sich im Griffteil 4 ein Sende- und Empfangsgerät, durch welches der Griffteil 4 mit der Steuer- und Datenverarbeitungseinheit 5 funktechnisch verbunden ist.

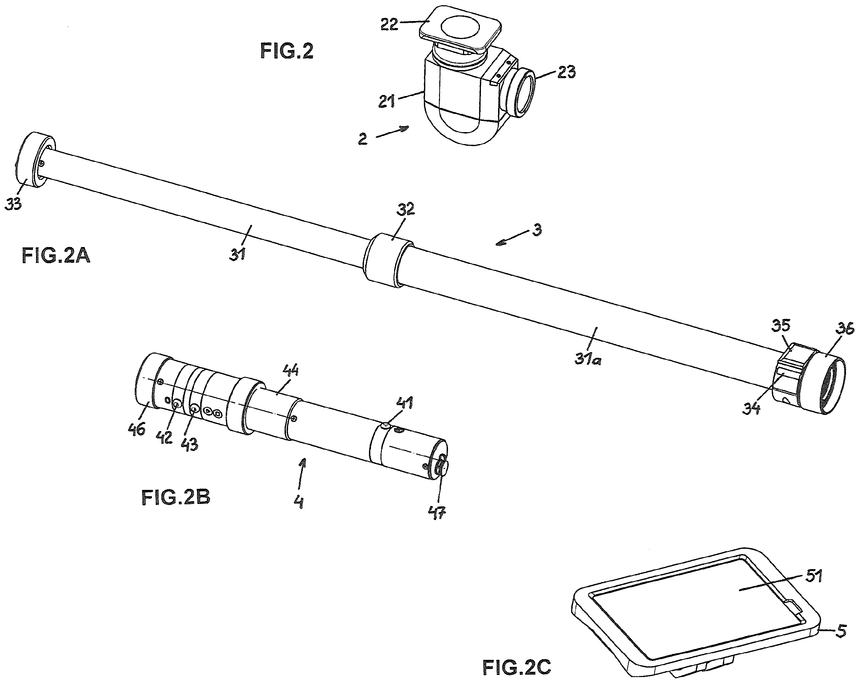

Wie dies aus FIG.2 ersichtlich ist, ist die Messeinrichtung 2 mit einem Gehäuse 21 ausgebildet, in welchem sich ein Feuchtigkeitssensor 22 befindet, welcher für die Durchführung der Messungen an die Unterseite des Siebbandes 11 angepresst wird. Weiters ist die Messeinrichtung 2 mit einem Kupplungsteil 23 zu dessen Verbindung mit dem Messstab 3 versehen.

Wie dies aus FIG.2A ersichtlich ist, besteht der Messstab 3 aus zwei Rohrstücken 31 und 31a, welche ineinander verschiebbar sind und welche mittels eines Klemmringes 32 in ihrer gegenseitigen Lage feststellbar sind, wodurch die Länge des Messstabes 3 einstellbar ist. An seinem linken Ende ist der Messstab 3 mit einem ersten Kupplungsteil 33 ausgebildet, welcher dem Kupplungsteil 23 der Messeinrichtung 2 zugeordnet ist. Weiters ist der Messstab 3 mit einer Wasserwaage 34 und mit einem digitalen Display 35 ausgebildet. An seinem rechten Ende ist der Messstab 3 mit einem zweiten Kupplungsteil 36 ausgebildet, welcher zur Verbindung des Messstabes 3 mit dem Griffteil 4 dient.

Wie dies aus FIG.2B ersichtlich ist, ist der Griffteil 4 mit einer Einschalttaste 41, mit einer Funktionstaste 42, mit einer Starttaste 43 und mit einem transparenten Bereich 44 mit einer LED-Leuchte ausgebildet. An seinem linken Endteil ist der Griffteil 4 mit einem ersten Kupplungsteil 46 ausgebildet, welcher dem Kupplungsteil 36 des Messstabes 3 zugeordnet ist. An seinem rechten Endteil ist der Griffteil 4 mit einer Ladebuchse 47 ausgebildet. Im Innenraum des Griffteiles 4 befinden sich elektrische und elektronische Geräte zur Durchführung der Messungen, wie ein Datenspeicher, ein Sende- und Empfangsgerät, Steuergeräte, Schaltgeräte u.dgl. sowie eine Batterie.

In FIG.2C ist die Steuer- und Datenspeichereinheit 5 dargestellt, welche mit einem Display 51 ausgebildet ist und welche gleichfalls mit einem Sende- und Empfangsgerät ausgebildet ist, welches mit dem im Griffteil 4 befindlichen Sende- und Empfangsgerät zusammenarbeitet.

Für die Durchführung von Messungen werden die Messeinrichtung 2, der Messstab 3 und der Griffteil 4 miteinander verbunden, wie dies in FIG.1 dargestellt ist und wird die Einrichtung 2 an die Unterseite des Siebbandes 11 angepresst.

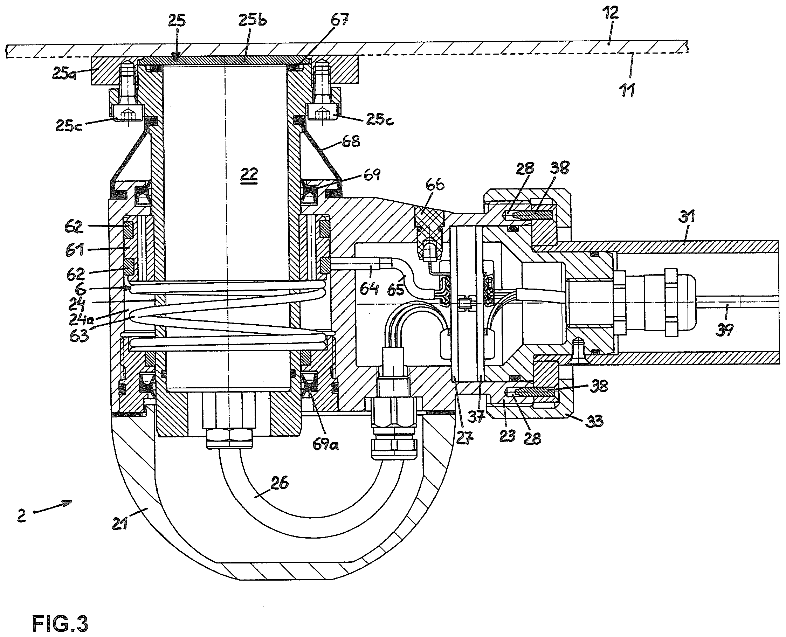

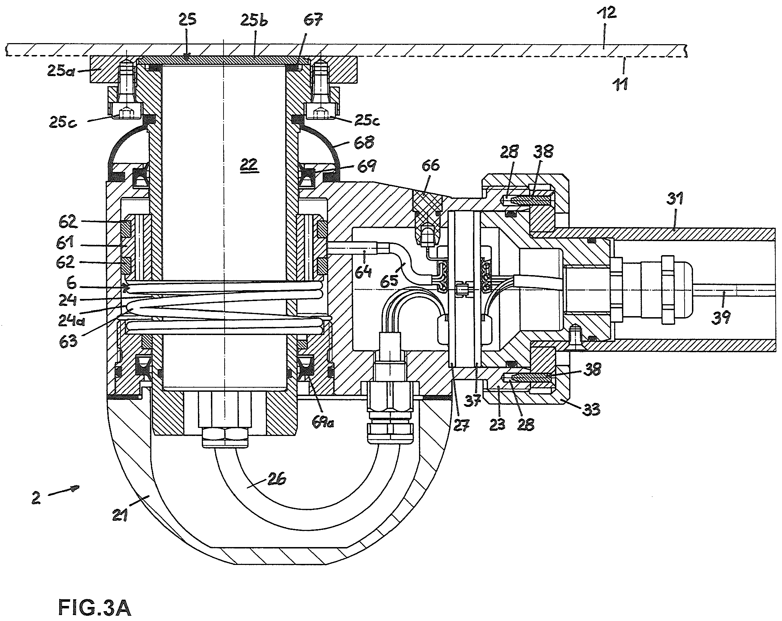

In FIG.3 ist die Einrichtung 2 zur Messung der Feuchtigkeit des auf dem Siebband 11 in einer Anlage zur Papiererzeugung befindlichen Pulpematerials 12 dargestellt. Die Einrichtung 2 ist mit dem Gehäuse 21 ausgebildet, welches einen zylindrischen Hohlraum 24 umschließt. In diesem Hohlraum 24 befindet sich der Sensor 22 zur Messung der Feuchtigkeit des Pulpematerials 12. Am oberen Ende des Sensors 22 befindet sich eine Messplatte 25, welche aus einem äußeren metallischen Ringteil 25a und einem inneren Teil 25b aus einem keramischen Material besteht. Der Ringteil 25a ist am Sensor 22 durch Schrauben 25c befestigt. Der innere Teil 25b ist mittels des Ringteiles 25a am Sensor 22 austauschbar befestigt. Die Ausbildung des inneren Teiles 25b, durch welche die Messungen beeinflusst werden, wird so gewählt, um spezifischen Anforderungen der Messungen zu entsprechen. Der Ausgang des Sensors 22 ist über eine Leitung 26 an eine Platine 27 geführt, welche sich im Kupplungsteil 23 befindet.

Zur Erzielung von genauen Ergebnissen der Messungen der Feuchtigkeit des Pulpematerials 12 ist es erforderlich, die Messeinrichtung 2 an das Siebband 11 mit einem Druck anzupressen, welcher sich in einem vorgegebenen Druckbereich befindet. Um dies zu gewährleisten, ist der Sensor 22 innerhalb des Hohlraumes 24 höhenverstellbar gelagert und ist die Messeinrichtung 2 mit einer Einrichtung 6 zur Erzielung eines Anpressdruckes innerhalb des vorgegebenen Bereiches ausgebildet. Der Hohlraum 24 ist mit einer im Querschnitt ringförmigen Erweiterung 24a ausgebildet. Am Sensor 22 ist eine Gleithülse 61 befestigt, welche mit zwei in axialem Abstand voneinander befindlichen Gleitringen 62 ausgebildet ist. Zwischen der Gleithülse 61 und der unteren Stirnfläche der Erweiterung 24a befindet sich ein Federelement 63 in Form einer Schraubendruckfeder, welche zwischen dem Gehäuse 21 und dem Sensor 22 zur Wirkung kommt. Sobald der Sensor 22 mit der Messplatte 25 an die Unterseite des Siebbandes 11 angepresst wird, wird er entgegen der Wirkung der Schraubendruckfeder 63 im Gehäuse 21 nach unten verschoben, wodurch die Schraubendruckfeder 63 zusammengepresst wird, wobei der von dieser erzeugte Druck, mit welchem der Sensor 22 an das Siebband 11 angepresst wird, ansteigt.

In FIG.3 ist die obere Endlage des Sensors 22 dargestellt, bei welcher die Messplatte 25 an das Siebband 11 mit nur geringem Druck anliegt. Demgegenüber ist in FIG.3A eine mittlere Höhenlage des Sensors 22 dargestellt, bei welcher durch die Schraubendruckfeder 63 ein mittlerer Druck ausgeübt wird, mit welchem der Sensor 22 an das Siebband 11 angepresst wird.

Um denjenigen Druck, mit welchem die Messplatte 25 an das Siebband 11 angepresst wird, zu erfassen und diesen in den Messergebnissen zu berücksichtigen, ist der Gleithülse 61 ein Lagesensor 64 zugeordnet. Die Gleithülse 61 ist aus einem Metall hergestellt. Demgegenüber sind die Gleitringe 62 aus einem Kunststoffmaterial hergestellt. Bei einer Verstellung der Gleithülse 61 und der Gleitringe 62 gegenüber dem Lagesensor 64 wird aufgrund der Änderung in der elektrischen Leitfähigkeit des diesem gegenüber liegenden Bereiches diejenige Lage erfasst, in welcher sich der Lagesensor 64 zwischen den beiden Gleitringen 62 befindet. In dieser Lage wird durch die Schraubendruckfeder 63 der vorgegebene Anpressdruck erzeugt. Der Ausgang des Lagesensors 64 ist über eine Leitung 65 an eine im Gehäuse 21 befindliche LED-Leuchte 66 geführt, durch welche die für den vorgegebenen Anpressdruck erforderliche Lage des Sensors 22 angezeigt wird. Da die Leitung 65 auch an die Platine 27 angeschlossen ist, wird der Anpressdruck auch an die Steuer- und Datenverarbeitungseinheit 5 übertragen.

Zwischen dem inneren Teil 25b der Messplatte 25 und dem Sensor 22 zur Messung der Feuchtigkeit der Papierpulpe 12 befindet sich ein Dichtungsring 67. Weiters befindet sich zwischen dem Sensor 22 und dem Gehäuse 21 eine Dichtungsmanschette 68. Zudem ist der Sensor 22 gegenüber dem Gehäuse 21 mittels weiteren Dichtungsringen 69 und 69a abgedichtet. Durch die Dichtungselemente 67, 68, 69, 69a wird verhindert, dass aus dem Pulpematerial 12 austretende Flüssigkeit in das Gehäuse 21 eindringt. Hierdurch wird die Einrichtung 6 zur Erzeugung des angestrebten Druckes, mit welchem der Sensor 22 an das Siebband 11 angepresst wird, gegenüber dem Eintritt von Flüssigkeit geschützt, wodurch sie nicht beschädigt wird bzw. funktionsfähig bleibt.

Zur Kupplung der Einrichtung 2 zur Messung der Feuchtigkeit mit dem Messstab 3 dienen einerseits der Kupplungsteil 23 und andererseits der Kupplungsteil 33 des Messstabes 3. Der Kupplungsteil 23 ist als Rohrstück ausgebildet, welches mit einem Außengewinde ausgebildet ist. Der Kupplungsteil 33 ist als Überwurfmutter ausgebildet, welche auf den Kupplungsteil 23 aufgeschraubt wird. Innerhalb dieser Kupplung ist der Platine 27 eine Platine 37 des Messstabes 3 zugeordnet. Diese beiden Platinen 27 und 37 sind mit einander zugeordneten Kontakten ausgebildet. Um bei der Kupplung des Messgerätes 2 und des Messstabes 3 die Platinen 27 und 37 in der richtigen Lage aneinander zur Anlage zubringen, ist der Kupplungsteil 23 mit Positionsbohrungen 28 ausgebildet und ist der Kupplungsteil 33 mit Positionsstiften 38 ausgebildet, welche den Positionsbohrungen 28 zugeordnet sind. An die Platine 37 ist eine wendelförmig ausgebildete Datenleitung 39 angeschlossen, welche sich innerhalb des Messstabes 3 befindet. Durch die am Messstab 3 befindliche Wasserwaage 34 kann die Winkellage der Messeinrichtung 2 gegenüber dem Siebband 11 überprüft werden. Durch das Display 35 werden ausgewählte Daten, welche für die Durchführung der Messungen von Relevanz sind, angezeigt.

Durch die im Griffteil 4 befindliche Einschalttaste 41 wird die gesamte Vorrichtung 1 zur Messung der Feuchtigkeit des am Siebband 11 befindlichen Pulpematerials 12 eingeschaltet. Durch die Funktionstaste 42 erfolgt eine Auswahl und Einstellung von Funktionen. Durch die Starttaste 43 werden dann, sobald der erforderliche Anpressdruck des Sensors 22 an das Siebband 11 erreicht ist, was mittels des Lagesensors 64 durch die LED-Leuchte 66 angezeigt wird, die Messungen der Feuchtigkeit des auf dem Siebband 11 befindlichen Pulpematerials 12 durchgeführt. Sämtliche Daten betreffend die Positionierungen der Messeinrichtung 2 und die Ergebnisse der Messungen werden in einem im Griffteil 4 befindlichen Zwischenspeicher gespeichert. Weiters werden diese Daten über das im Griffteil 4 befindliche Sendegerät an die Steuer- und Datenverarbeitungseinheit 5 übertragen, in welcher sie gespeichert und ausgewertet werden, wobei sie am Display 51 angezeigt werden können.

Diese Vorrichtung wird wie folgt zum Einsatz gebracht:
In der Steuer- und Datenverarbeitungseinheit 5 werden die konstruktiven Merkmale einer Anlage zur Papiererzeugung geladen und die Anlage wird am Display 51 angezeigt. Weiters werden die für den Betrieb der Anlage maßgeblichen Daten, wie die Geschwindigkeit, mit welcher das Siebband 11 bewegt wird, und die Parameter des Papiers, welches erzeugt wird, in die Steuer- und Datenverarbeitungsanlage 5 eingespeichert. In der Folge werden diejenigen Stellen, an welchen Messungen der Feuchtigkeit vorgenommen werden sollen, eingespeichert, wobei diese Messstellen gleichfalls am Display 51 angezeigt werden. Weiters wird ein Messprogramm eingegeben, welches u.a. darin besteht, ob vor Beginn der Messungen eine Kalibrierung durchgeführt wird. Hierauf wird die Messeinrichtung 2 an den einzelnen Messstellen an das Siebband 11 angepresst. Sobald sich derjenige Druck, mit welchem die Messeinrichtung 2 an das Siebband 11 angedrückt wird, im vorgegebenen Bereich befindet, werden die Messungen der Feuchtigkeit des Pulpematerials 12 vorgenommen. Die dadurch gewonnenen Messwerte werden den einzelnen Messstellen zugeordnet und gespeichert. In der Folge werden die Messergebnisse ausgewertet und werden sie für die Steuerung des Produktionsvorganges herangezogen.

## Patentansprüche

1. Messstab (3) mit einer Einrichtung (2) zur Messung der Feuchtigkeit des auf einem Siebband (11) in einer Anlage zur Erzeugung von Papier befindlichen Pulpematerials (12), welche einen Feuchtigkeitssensor (22) aufweist, mit einer dieser zugeordneten Steuer- und Datenverarbeitungseinheit (5) sowie mit einem Sende- und Empfangsgerät und bzw. oder mit einem Anschluss für eine Datenleitung zur Übermittlung der Messdaten an die Steuer- und Datenverarbeitungseinheit (5), **dadurch gekennzeichnet, dass** die Einrichtung (2) zur Messung der Feuchtigkeit des Pulpematerials mit einem Messgerät (6) zur Ermittlung und Anzeige desjenigen Druckbereiches, mit welchem sie an das Siebband (11) angepresst wird, ausgebildet ist.

2. Messstab (3) nach Anspruch 1, **dadurch gekennzeichnet, dass** die Einrichtung (2) zur Messung der Feuchtigkeit mit einem Gehäuse (21) ausgebildet ist, in welchem der Feuchtigkeitssensor (22), welcher unter Wirkung eines Federelementes (63) steht, verstellbar ist.

3. Messstab (3) nach Anspruch 2, **dadurch gekennzeichnet, dass** das Gehäuse (21) mit einem zylindrischen Hohlraum (24) ausgebildet ist, in welchem sich der Feuchtigkeitssensor (22) befindet und in welchem er unter Wirkung des Federelementes (63) verschiebbar ist.

4. Messstab (3) nach Anspruch 3, **dadurch gekennzeichnet, dass** das Federelement (63) durch eine Schraubendruckfeder gebildet ist, welche zwischen dem Feuchtigkeitssensor (22) und dem Gehäuse (21) wirksam ist.

5. Messstab (3) nach einem der Ansprüche 3 und 4, **dadurch gekennzeichnet, dass** der im Gehäuse (21) befindliche zylindrische Hohlraum (24) mit einem im Querschnitt ringförmigen Bereich (24a) ausgebildet ist und dass der Feuchtigkeitssensor (22) mit einer an diesem befestigten Gleithülse (61) ausgebildet ist, welche in den ringförmigen Bereich (24a) hineinragt und zwischen dessen Stirnflächen verschiebbar ist.

6. Messstab (3) nach Anspruch 5, **dadurch gekennzeichnet, dass** sich das Federelement (63) zwischen der Gleithülse (61) und dem Gehäuse (21) befindet.

7. Messstab (3) nach einem der Ansprüche 1 bis 6, **dadurch gekennzeichnet, dass** der Feuchtigkeitssensor (22) an seiner dem Siebband (11) zugewandten Seite mit einer verschleißfesten Auflage (25), insbesondere einer Platte aus einem keramischen Material, ausgebildet ist.

8. Messstab (3) nach Anspruch 7, **dadurch gekennzeichnet, dass** die verschleißfeste Auflage (2) am Feuchtigkeitssensor (22) auswechselbar befestigt ist.

9. Messstab (3) nach Anspruch 8, **dadurch gekennzeichnet, dass** zwischen der Auflage (25) aus keramischem Material und dem Feuchtigkeitssensor (22) eine Dichtung (67) vorgesehen ist.

10. Messstab (3) nach einem der Ansprüche 2 bis 9, **dadurch gekennzeichnet, dass** der Feuchtigkeitssensor (22) und das Gehäuse (21) mittels einer elastischen Dichtungsmanschette (68) und gegebenenfalls mittels mindestens eines Dichtungsringes (69, 69a) gegenüber dem Eintritt von Flüssigkeit abgedichtet sind.

11. Messstab (3) nach einem der Ansprüche 5 bis 10, **dadurch gekennzeichnet, dass** der Gleithülse (61) mindestens ein Lagesensor (64) zugeordnet ist, durch welchen die Stellung des Feuchtigkeitssensors (22) gegenüber dem Gehäuse (21) ermittelt und angezeigt wird.

12. Messstab (3) nach einem der Ansprüche 5 bis 11, **dadurch gekennzeichnet, dass** die Gleithülse (61) mit zwei voneinander in axialem Abstand befindlichen Gleitringen (62) ausgebildet ist, welche mit dem Lagesensor (64) zusammenwirken.

13. Messstab (3) nach Anspruch 12, **dadurch gekennzeichnet, dass** an den Lagesensor (64) eine im Gehäuse (21) befindliche LED-Leuchte (66) angeschlossen ist, durch welche ein Lichtsignal abgegeben wird, sobald sich durch eine Verschiebung des Feuchtigkeitssensors (22) gegenüber dem Gehäuse (21) der Lagesensor (64) in dem zwischen den Gleitringen (62) befindlichen Bereich befindet.

14. Messstab (3) nach einem der Ansprüche 2 bis 12, **dadurch gekennzeichnet, dass** das Gehäuse (21) der Messeinrichtung (2) und der Messstab (3) mit einander zugeordneten Kupplungsteilen (23, 33) zu deren mechanischer und elektrischer Verbindung ausgebildet sind.

15. Messstab (3) nach Anspruch 14, **dadurch gekennzeichnet, dass** die Kupplungsteilen (23, 33) mit einander zugeordneten Platinen (27, 37) und Zentrierelementen (28, 38) ausgebildet sind.

16. Messstab (3) nach einem der Ansprüche 1 bis 15, **dadurch gekennzeichnet, dass** der Messstab (3) in seiner Länge ein- und feststellbar ist.

## Claims

1. Measuring rod (3) comprising a device (2), which has a moisture sensor (22), for measuring the moisture of the pulp material (12) located on a sieve belt (11) in an installation for producing paper, a control and data processing unit (5) assigned thereto, and a transmitting and receiving unit and/or a port for a data cable for transmitting the measurement data to the control and data processing unit (5), **characterized in that** the device (2) for measuring the moisture of the pulp material is configured with a measuring unit (6) for determining and displaying the pressure range with which it is pressed against the sieve belt (11).

2. Measuring rod (3) according to claim 1, **characterized in that** the device (2) for measuring the moisture is configured with a housing (21), in which the moisture sensor (22), which is under the effect of a spring element (63), is movable.

3. Measuring rod (3) according to claim 2, **characterized in that** the housing (21) is configured with a cylindrical cavity (24), in which the moisture sensor (22) is located and in which the latter is movable under the effect of the spring element (63).

4. Measuring rod (3) according to claim 3, **characterized in that** the spring element (63) is formed by a helical compression spring which acts between the moisture sensor (22) and the housing (21).

5. Measuring rod (3) according to one of claims 3 and 4, **characterized in that** the cylindrical cavity (24) located in the housing (21) is configured with a region (24a) which is ring-shaped in cross-section, and **in that** the moisture sensor (22) is configured with a sliding sleeve (61) fastened thereto, which projects into the ring-shaped region (24a) and is movable between the end faces thereof.

6. Measuring rod (3) according to claim 5, **characterized in that** the spring element (63) is located between the sliding sleeve (61) and the housing (21).

7. Measuring rod (3) according to one of claims 1 to 6, **characterized in that** the moisture sensor (22) is configured with a wear-resistant support (25), in particular a plate made of a ceramic material, on the side thereof facing towards the sieve belt (11).

8. Measuring rod (3) according to claim 7, **characterized in that** the wear-resistant support (2) is fastened to the moisture sensor (22) in a replaceable manner.

9. Measuring rod (3) according to claim 8, **characterized in that** a seal (67) is provided between the support (25) made of ceramic material and the moisture sensor (22).

10. Measuring rod (3) according to one of claims 2 to 9, **characterized in that** the moisture sensor (22) and the housing (21) are sealed against the ingress of liquid by means of an elastic sealing cuff (68) and optionally by means of at least one sealing ring (69, 69a).

11. Measuring rod (3) according to one of claims 5 to 10, **characterized in that** at least one position sensor (64), by which the position of the moisture sensor (22) relative to the housing (21) is determined and displayed is assigned to the sliding sleeve (61).

12. Measuring rod (3) according to one of claims 5 to 11, **characterized in that** the sliding sleeve (61) is configured with two sliding rings (62) which are located at an axial spacing from one another and which cooperate with the position sensor (64).

13. Measuring rod (3) according to claim 12, **characterized in that** connected to the position sensor (64) is an LED lamp (66) which is located in the housing (21) and by which a light signal is output as soon as the position sensor (64) is located in the region between the sliding rings (62) due to a displacement of the moisture sensor (22) relative to the housing (21).

14. Measuring rod (3) according to one of claims 2 to 12, **characterized in that** the housing (21) of the measuring device (2) and the measuring rod (3) are configured with mutually associated coupling parts (23, 33) for the mechanical and electrical connection thereof.

15. Measuring rod (3) according to claim 14, **characterized in that** the coupling parts (23, 33) are configured with mutually associated circuit boards (27, 37) and centring elements (28, 38).

16. Measuring rod (3) according to one of claims 1 to 15, **characterized in that** the length of the measuring rod (3) is adjustable and settable.

## Revendications

1. Palpeur de mesure (3) avec un dispositif (2) pour la mesure de l'humidité d'une matière en pulpe (12) qui se trouve sur une bande de tamis (11) dans une installation pour la production de papier, qui présente un capteur d'humidité (22), avec une unité de commande et de traitement de données (5) associée à celui-ci et avec un émetteur-récepteur et/ou avec une connexion pour une ligne de données pour la transmission des données de mesure à l'unité de commande et de traitement de données (5), **caractérisé en ce que** le dispositif (2) pour la mesure de l'humidité de la matière en pulpe est muni d'un appareil de mesure (6) pour déterminer et indiquer la plage de valeurs de pression dans laquelle il est pressé sur la bande de tamis (11).

2. Palpeur de mesure (3) selon la revendication 1, **caractérisé en ce que** le dispositif (2) pour la mesure de l'humidité est muni d'un boîtier (21) dans lequel le capteur d'humidité (22), qui est soumis à l'action d'un élément de ressort (63), peut être déplacé.

3. Palpeur de mesure (3) selon la revendication 2, **caractérisé en ce que** le boîtier (21) est muni d'une cavité cylindrique (24) dans laquelle se trouve le capteur d'humidité (22) et dans laquelle il peut coulisser sous l'action de l'élément de ressort (63).

4. Palpeur de mesure (3) selon la revendication 3, **caractérisé en ce que** l'élément de ressort (63) est formé par un ressort hélicoïdal de compression qui agit entre le capteur d'humidité (22) et le boîtier (21).

5. Palpeur de mesure (3) selon l'une des revendications 3 et 4, **caractérisé en ce que** la cavité cylindrique (24) du boîtier (21) est munie d'une zone de section annulaire (24a) et **en ce que** le capteur d'humidité (22) est muni d'une douille coulissante (61) qui lui est fixée, qui dépasse dans la zone annulaire (24a) et peut coulisser entre les faces d'extrémité de celle-ci.

6. Palpeur de mesure (3) selon la revendication 5, **caractérisé en ce que** l'élément de ressort (63) se trouve entre la douille coulissante (61) et le boîtier (21).

7. Palpeur de mesure (3) selon l'une des revendications 1 à 6, **caractérisé en ce que** le capteur d'humidité (22) est muni, sur son côté tourné vers la bande de tamis (11), d'une surface d'appui résistante à l'abrasion (25), en particulier d'une plaque de matériau céramique.

8. Palpeur de mesure (3) selon la revendication 7, **caractérisé en ce que** la surface d'appui résistante à l'abrasion (2) est fixée sur le capteur d'humidité (22) de façon interchangeable.

9. Palpeur de mesure (3) selon la revendication 8, **caractérisé en ce qu'**un joint (67) est prévu entre la surface d'appui (25) en matériau céramique et le capteur d'humidité (22).

10. Palpeur de mesure (3) selon l'une des revendications 2 à 9, **caractérisé en ce que** le capteur d'humidité (22) et le boîtier (21) sont rendus étanches à la pénétration de liquide au moyen d'un manchon d'étanchéité élastique (68) et éventuellement au moyen d'un joint d'étanchéité (69, 69a).

11. Palpeur de mesure (3) selon l'une des revendications 5 à 10, **caractérisé en ce que** la douille coulissante (61) est associée à au moins un capteur de position (64) qui détermine et indique la position du capteur d'humidité (22) par rapport au boîtier (21).

12. Palpeur de mesure (3) selon l'une des revendications 5 à 11, **caractérisé en ce que** la douille coulissante (61) est munie de deux bagues coulissantes (62) espacées d'une distance axiale, qui coopèrent avec le capteur de position (64).

13. Palpeur de mesure (3) selon la revendication 12, **caractérisé en ce qu'**une lampe à DEL (66) située dans le boîtier (21) est connectée au capteur de position (64) et émet un signal lumineux dès qu'une translation du capteur d'humidité (22) par rapport au boîtier (21) du capteur de position (64) se trouve dans la zone comprise entre les bagues coulissantes (62).

14. Palpeur de mesure (3) selon l'une des revendications 2 à 12, **caractérisé en ce que** le boîtier (21) du dispositif de mesure (2) et le palpeur de mesure (3) sont munis de parties d'accouplement (23, 33) associées les unes aux autres pour leur liaison mécanique et électrique.

15. Palpeur de mesure (3) selon la revendication 14, **caractérisé en ce que** les parties d'accouplement (23, 33) sont munies de platines (27, 37) et d'éléments de centrage (28, 38) associés les uns aux autres.

16. Palpeur de mesure (3) selon l'une des revendications 1 à 15, **caractérisé en ce que** la longueur du palpeur de mesure (3) peut être ajustée et fixée.
